# EUROPEAN PATENT SPECIFICATION

(11) **EP 0 940 132 B1**
(45) Date of publication and mention of the grant of the patent: **19.11.2003**
(21) Application number: 99104447.0
(22) Date of filing: 05.03.1999
(51) Int. Cl.: A61F 13/15

(54) **Absorbent article with flaps capable of gathering the undergarment**
Absorbierender Artikel dessen Flügel fähig sind, die Unterwäsche zu umschliessen
Article absorbant pourvu de rabats capables d'envelopper partiellement le sous-vêtement

(30) Priority: 06.03.1998 US 36717
(43) Date of publication of application: 08.09.1999
(73) Proprietor: McNeil-PPC, INC., Skillman, NJ 08558 (US)
(72) Inventor: Mavinkurve, Pramod S., Princeton, NJ 08540 (US); Glasgow, Tara, Yardley, PA 19067 (US)
(74) Representative: Groening, Hans Wilhelm, Dipl.-Ing.

(56) References cited:
- WO-A-94/12135
- WO-A-95/20932
- WO-A-97/00655
- US-A- 5 391 162

## Description

### FIELD OF THE INVENTION

The present invention relates to structures designed for absorbing body exudate, and more particularly, to an improved sanitary absorbent article having an enhanced capability to protect the undergarment from wetting.

### BACKGROUND OF THE INVENTION

A sanitary napkin typically comprises an elongated main body that is intended to be placed in the crotch portion of the undergarment so it remains in contact with the perineal region of the wearer. The sanitary napkin includes a liquid-permeable cover layer located over an absorbent core that is designed to collect and store body exudate discharged by the user and a liquid-impermeable barrier layer located under the absorbent core.

Sanitary napkins have been found to frequently experience leakage along the longitudinal sides of the napkin. In an attempt to reduce the possibility of undergarment or garment wetting resulting from side failures, several solutions have been proposed by the industry. One attempt to solve this problem is to provide the main body of the sanitary napkin with flaps that are folded about the edges of the undergarment to form upstanding walls which provide a shielding function. These flaps originate from the side edges of the main body and are provided with adhesive zones allowing the flaps to be secured against the outside surface of the undergarment. Thus, in the event that menstrual fluid leaks past the side edges of the main absorbent body of the napkin, the undergarment will be protected from the fluid by virtue of the flaps.

This approach, however, presents a number of drawbacks. For example, in an attempt to provide enhanced protection against side leakage, manufacturers have substantially increased the width of the flaps, (as measured along the length of the napkin) to cover as much of the undergarment as possible- Major difficulties observed with sanitary napkins provided with such wide flaps are the stresses created when fitting the flap about the curved edges of an undergarment and the inability of the flaps to conform well to the edges of the undergarment. Since the edges of the undergarment are outwardly curved from the narrow central crotch portion to become wider as the undergarment encircles the legs, wide flaps, when folded about the edges of the crotch portion of the undergarment will have a tendency to detach and/or form wrinkles which cause irritation and discomfort by chafing the inner thighs of the wearer and are also detrimental to the adhesive bond between the flaps and the undergarment, resulting in a further likelihood of detachment of the flaps from the undergarment. One possibility which has been proposed to solve this problem is to use a longitudinally extensible or elastic material to form wide flaps which are more comfortable to the shape of the undergarment edges.

Under a different approach, the main body of the sanitary napkin is provided with flaps that are affixed to the barrier layer, i.e. on the garment facing side of the napkin, at a point located inwardly of the respective side edges of the sanitary napkin. The flaps may be adhered to the underside of the wearer's undergarment or may be sufficiently long so they completely encircle the crotch portion of the undergarment and they are retained to one another in overlapping relationship. Since the flaps originate inwardly of the respective side edges, they have the effect of gathering the undergarment so that it remains inward of the longitudinal side edges of the main body. Thus, it is the body-facing side of the main absorbent body that provides the primary undergarment shielding function while the flaps positively prevent the edges of the undergarment from extending over the cover layer of the main body where they are subject to wetting. Examples of sanitary napkins constructed in accordance with this concept are described in U.S. patent 4,900,320 granted to McNeil-PPC on February 13, 1990 and in co-pending patent application, U. S. Serial Number 08/772,343, (PCT/CA97/00990 filed December 22, 1997).

WO-A-9412135 and US-A-5391162 disclose a sanitary absorbent article comprising a main body having longitudinal side edges, a body-facing fluid-permeable upper layer, a garment-facing fluid-impervious lower layer, and an absorbent layer between the upper layer and the lower layer; two panels extending from the longitudinal side edges, including a proximal end region and a distal end region, and an juncture zone which attaches a portion of the panel to a barrier layer inwardly from the longitudinal side edge of the main body, thereby defining a freely extending flap; a looping member defined by the portion of the body portion of the panel between the proximal end and the juncture zone, the flap being capable of being folded about an edge of an undergarment in a crotch portion thereof; whereby, when the portion of the flap is in a folded condition, the flap provides a means for holding the edge of the undergarment inwardly of said longitudinal side edge.

WO-A-9520932 and WO-A-9700655 disclose sanitary absorbent articles having flaps provided with a stiffening element.

### OBJECTS AND STATEMENT OF THE INVENTION

An object of the invention is to provide a sanitary napkin that is capable of protecting the undergarment of the wearer against wetting.

In accordance with the invention, there has been provided a sanitary absorbent article adapted to be worn in a crotch portion of an undergarment, according to claim 1.

Preferred embodiments of the invention are described in dependent claims 2 to 9.

The absorbent structure is generally an absorbent pulp fluff material, and may optionally comprise a dual-layer structure, including a highly porous transfer layer on top of a sphagnum moss absorbent core. It will be appreciated, however, that different absorbent systems can be used without departing from the spirit of the invention.

The panels originate from a central portion of the longitudinal side edges of the main body. Each panel has a base portion located at a proximal end that is continuous with and adjacent to the respective side edge of the main body, and flap portion a distal end continuous with the proximal end and which extends laterally outward from the longitudinal sides of the absorbent article. The base portion has a length longer than the flap portion. The distal end of the panel is folded underneath the barrier layer, i.e. on the garment facing surface of the absorbent article, and the panel is affixed to the barrier layer along the base portion at a juncture zone which is adjacent to the proximal end and inward of the longitudinal sides of the absorbent article so it remains in a folded condition. The folded portion of the flap forms a looping member which, when affixed to the barrier adjacent the flange, forms a first liquid barrier along the longitudinal sides of the napkin. The distal end thus forms a flap which extends inwardly from the respective side edge toward the longitudinal centerline of the absorbent article and is affixed inwardly of the side edge. As a result, when the flaps are folded about the crotch portion of the undergarment, they maintain or gather the undergarment sufficiently so the portions of the side edges of the undergarment located in the central area of the main body (those portions of the side edges of the undergarment are most susceptible of being wetted if failure occurs) remain within a boundary defined by the longitudinal side edges of the absorbent article. Accordingly, the main body of the absorbent article provides the major shielding function and protects the undergarment from being wetted and soiled with menstrual liquid.

The portion of the panel between the proximal end and the juncture zone defines a looping member. In order to reduce the cost of manufacturing the article, the looping member may be substantially unitized. In this context the term "substantial unitized" is used to indicate that the layers forming the looping member and all other material contained therein are attached together over an appreciable portion thereof to form an integral whole. The presence of unattached or free-floating structures within the looping member would significantly increase the cost of manufacture of the article.

Another advantage of this sanitary napkin configuration is the ability of the longitudinal side portions of the main body to resist the tendency to slope downwardly due to tension imparted by the flaps. That is, since the body portion of each flap originates inwardly of the respective side edge of the absorbent article, any tension which arises when the flap is fastened to the undergarment, will act against the barrier layer inwardly of the side edge. As a consequence, the peripheral edge portions of the main body are less likely to slope downwardly by comparison to a design where the tension imparted by the flaps act solely on the side edges. As mentioned before, such downward sloping is undesirable because it permits liquid pooling on the cover layer to leak sideways under the effect of gravity.

Further described herein is a sanitary napkin, comprising:
(A) a main body having opposed longitudinal side edges, said main body including:
   (i) a liquid-permeable cover layer,
   (ii) an absorbent layer underneath the liquid-permeable cover layer, and
   (iii) a liquid-impervious barrier layer below the absorbent layer, the cover layer and barrier layer being sealed about a peripheral edge margin to enclose the absorbent layer;
(B) a longitudinally extending looping member extending laterally outward from each respective longitudinal side edge, each looping member integrally formed from either the cover layer, the barrier layer, or both the cover layer and the barrier layer, the looping member being formed by folding at least one of said layers around and affixing the layer to the barrier layer at a juncture zone located inwardly of the respective longitudinal side edge;
(C) a pair of flaps on opposite sides of said main body, said flaps integrally formed from the looping member, said flaps being affixed to the barrier layer inwardly of the respective longitudinal side edge of the main body and projecting laterally from said main body in a direction generally transverse to the longitudinal side edges, each of said flaps including:
   (I) a distal end continuous with the respective juncture zone which attaches at least a portion of said flap to the barrier layer, the distal end being folded under the barrier layer and extending laterally inwardly in a direction away from the respective longitudinal side edge,
   (ii) a body portion retained to the distal end, the body portion being capable of being folded about a crotch portion of an undergarment on which the sanitary napkin is placed.
   (iii) stiffening elements located in the body portion of the flap which are capable of resisting deformation due to lateral compressive forces to the flap.

### BRIEF DESCRIPTION OF THE DRAWINGS

Figure 1 is a top plan view of a sanitary napkin;
Figure 2 is a bottom plan view of a sanitary napkin;
Figure 3 is a cross-sectional view taken along line 3-3 in Figure 1;
Figure 4 is a cross-sectional view of another embodiment of a sanitary napkin taken along line 3-3 in Figure 1;
Figure 5 is a cross-sectional view of another embodiment of a sanitary napkin taken along line 3-3 in Figure 1;
Figure 6 is a cross-sectional view of another embodiment of a sanitary napkin taken along line 3-3 in Figure 1;
Figure 7 is a bottom plan view of another embodiment of a sanitary napkin;
Figure 8 is a bottom plan view of another embodiment of a sanitary napkin;
Figure 9 is a bottom plan view of another embodiment of a sanitary napkin;
Figure 10 is a perspective view of the sanitary napkin when placed in an undergarment;
Figure 11 is a cross-sectional view of the sanitary napkin when placed in an undergarment;
Figure 12 is a cross-sectional view of another embodiment of a sanitary napkin taken along line 3-3 in Figure 1;
Figure 13 is a cross-sectional view of another embodiment of a sanitary napkin taken along line 3-3 in Figure 1;

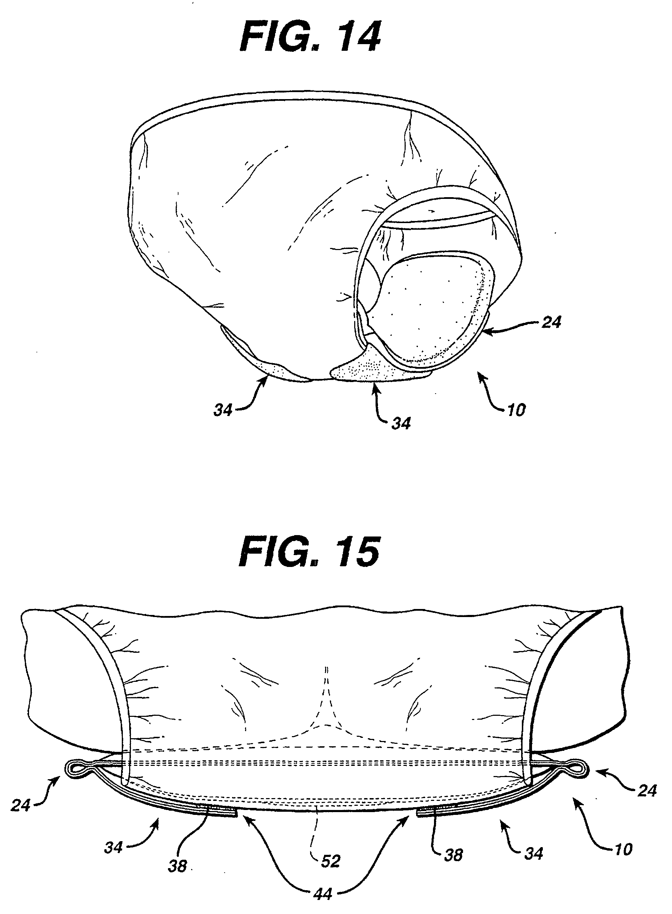

Figure 14 is a perspective view of the sanitary napkin when placed in an undergarment; and
Figure 15 is a cross-sectional view of the sanitary napkin when placed in an undergarment;
Figure 16 is a bottom plan view of an embodiment of a sanitary napkin constructed in accordance with the present invention;
Figure 17 is a top plan view of a flap having a stiffened portion in the form of an embossing pattern;
Figure 18 is a top plan view of a flap having a stiffened portion in the form of another embossing pattern;
Figure 19 is a top plan view of a flap having a stiffened portion in the form of another embossing pattern; and
Figure 20 is a top plan view of a flap having a stiffened portion in the form of another embossing pattern.

### DESCRIPTION OF A PREFERRED EMBODIMENT

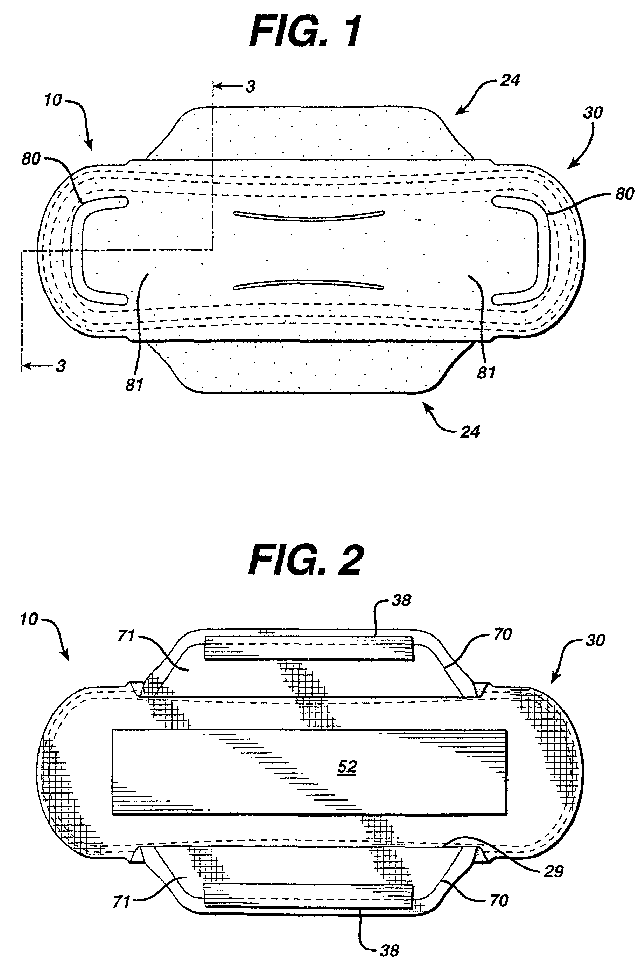

Referring now to the annexed drawings, more particularly to Figures 1, 2, and 3, there is shown a sanitary napkin which is designated comprehensively by the reference numeral 10 and which is characterized by the ability to better protect the undergarment of the wearer against wetting and soiling by menstrual liquid.

More specifically, the sanitary napkin 10 comprises a liquid-permeable cover layer 12 overlaying an absorbent system 14. The cover layer may be a relatively low density, bulky, high-loft non-woven web material. The cover layer 12 may be composed of only one type of fiber, such as polyester, or it may be composed of bicomponent fibers having a low melting point component and a high melting point component. The components of bicomponent fibers may be arranged with respect to each other as side by side or one surrounding another as a sheath around a core.
Examples of low and high melting components are polyethylene and polyester, polypropylene and polyester, polyethylene and high melting polyester. The use of appropriate bicomponent materials results in a fusible non-woven fabric. Examples of such fusible fabrics are described in U.S. patent 4,555,430 issued November 26, 1985 to Mays. Using a fusible fabric increases the ease with which the cover layer may be adhered to the adjacent transfer layer and/or to the barrier layer. Fibers may also be selected from a variety of natural and synthetic materials such as nylon, polyester, rayon (in combination with other fibers), cotton acrylic fiber and the like and combinations thereof.

The cover layer preferably has a relatively high degree of wettability, although the individual fibers comprising the cover may not be particularly hydrophilic. The cover material should also contain a great number of relatively large pores to absorb body fluid rapidly and transport it away from the body and the point of deposition. Preferably, the fibers which make up the cover layer should not lose their physical properties when they are wetted, in other words they should not collapse or lose their resiliency when subjected to water or body fluid. The cover may be treated, e.g. with surfactant and/or high energy discharge, e.g., corona discharge, to allow fluid to pass through it readily. Since the cover layer also functions to transfer the fluid quickly to the other layers of the absorbent structure, the cover is preferably wettable, hydrophilic and porous. Thus, when composed of synthetic hydrophobic fibers such as polyester or bicomponent fibers, the cover may be treated with a surfactant to impart the desired degree of wettability.

Alternatively, the cover layer 12 may be made of an apertured polymeric film. Because of the hydrophobicity and high porosity of apertured polymeric films, they quickly transfer body fluids deposited on the cover to the inner layers of the absorbent structure. Apertured films made of co-extruded polymers, examples of such being the RETICULON brand, which are described in U.S. Patent 4,690,679 are useful as cover layers in the absorbent structures of this invention.

The cover layer 12 may be micro or macro embossed to improve the texture of the polymeric film and reduce the plastic "feel" of the cover against a wearer's skin. Cover layer may be optionally adhered to the lower absorbent layer to further enhance fluid transfer from the cover to the next layer.

Adjacent to the cover layer 12 on its inner side and bonded to the cover layer 12 is an absorbent system 14 which comprises an optional fluid transfer layer 16 and an absorbent core 18 which together form the absorbent system 14. The transfer layer 16 provides the means of quickly receiving body fluid from the cover layer 12 and holding it until slower absorbing absorbent core 14 has an opportunity to acquire the fluid. The transfer layer 16 is, preferably, more dense than and has a larger proportion of smaller pores than the cover layer 12. These attributes allow the transfer layer 16 to contain body fluid and hold it away from the outer side of the cover layer 12, thereby preventing the fluid from re-wetting the cover layer 12 and its surface. However, the transfer layer is, preferably, not so dense as to prevent the rapid passage of the fluid through the layer into the absorbent core.

Transfer layer 16 generally comprises fibrous materials, such as wood pulp, polyester, rayon, flexible foam, or the like, or combinations thereof. The transfer layer 16 may also comprise thermoplastic fibers for the purpose of stabilizing the layer and maintaining its structural integrity. Transfer layer 16 may be treated with surfactant on one or both sides in order to increase its wettability, although generally the transfer layer 16 is relatively hydrophilic and may not require treatment. Transfer layer 16 is preferably bonded on both sides to the adjacent layers, i.e. the upper cover layer 12 and the lower absorbent core 18.

Immediately adjacent to and bonded to transfer layer 16 is absorbent core 18. Absorbent core 18 is preferably a highly dense layer having a fine porosity. It has a large liquid holding capacity and it is extremely retentive. Absorbent core 18 may be generally rectangular having substantially straight parallel side margins, or may be contoured to adapt to the body of the wearer such as in an hourglass shape or a dog-bone shape. In addition, core 18 may contain one or more embossed channels which stabilize the absorbent article and enhance fluid transfer within the absorbent core by capillary action. In one embodiment, the absorbent core 18 comprises a cellulosic pulp fluff material. In another embodiment, the absorbent core 18 comprises a compressed sphagnum moss material. In yet another embodiment, the absorbent core 18 comprises a combination of a cellulosic pulp fluff material and a compressed sphagnum moss material. In accordance a preferred embodiment, a compressed sphagnum moss material is formed as a board by air or wet laying and calendering to obtain a relatively thin, i.e., from about 0.01 to 0.10 inch thick, relatively dense, i.e., from about 0.2 to 1.0 g/cm³ sheet like structure. The structure may include a layer of Kraft tissue laminated on one or both surfaces of the sphagnum moss layer. Preferably, a fibrous component is admixed with the sphagnum moss material. The fibrous component is suitably a natural or synthetic textile fiber such as rayon, polyester, nylon, acrylic or the like, having a length of from about 7,62 to 3,81 cm (0.30 to 1 .5 inches) and a denier of from about 1.0 to 5. The fibrous component may be present in an amount from about 2 to 20% by weight, most preferably from 4 to 8%. Absorbent core 18 may also comprise other components such as wood pulp, synthetic wood pulp, thermomechanical pulp, mechanically ground pulp, polymers, surfactants, conjugate fibers, fusible fibers, binders, sphagnum moss particles, deodorants, superabsorbents, and the like and combinations thereof.

Underlying absorbent system 14 is a barrier layer 20 comprising liquid-impervious film material so as to prevent liquid that is entrapped in the absorbent core from egressing the sanitary napkin and staining the wearer's undergarment. Most preferably, the barrier layer 20 is made of polymeric film, such as co-extruded EVA/polyethylene laminate which is both inexpensive and readily available. The film is capable of fully blocking the passage of liquid or gas that may emanate from the absorbent system 14. In a variant, breathable films may be used that allow passage of gases while blocking liquid.

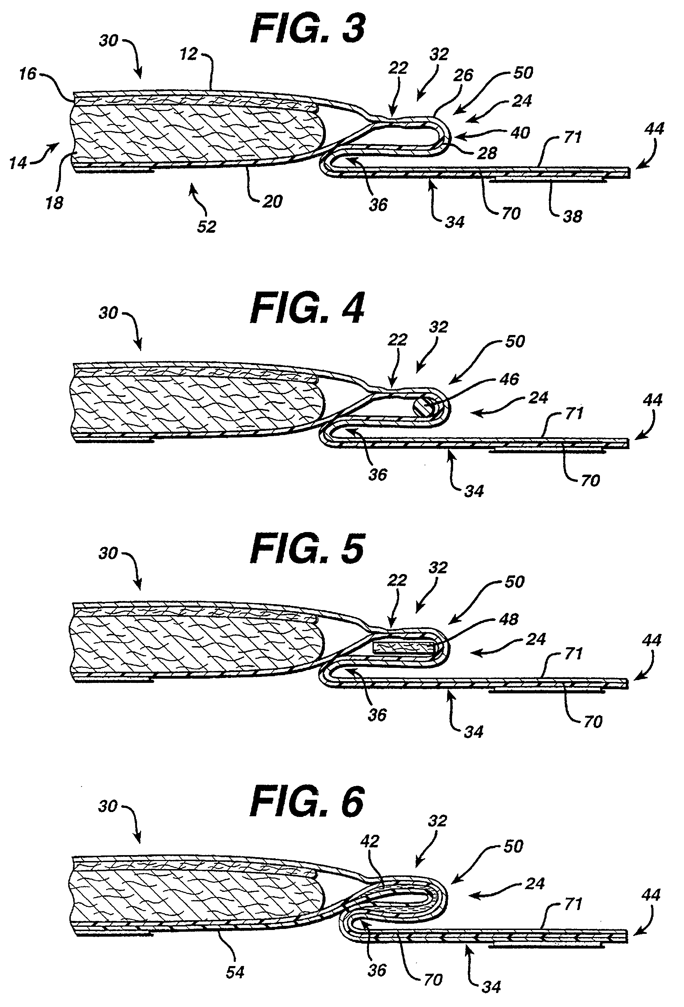

Cover layer 12 and barrier layer 20 are joined along their marginal edge portions to form a peripheral seal line or flange 22 as illustrated in Figures 3 - 6 which encloses and maintains the absorbent system 14 captive.

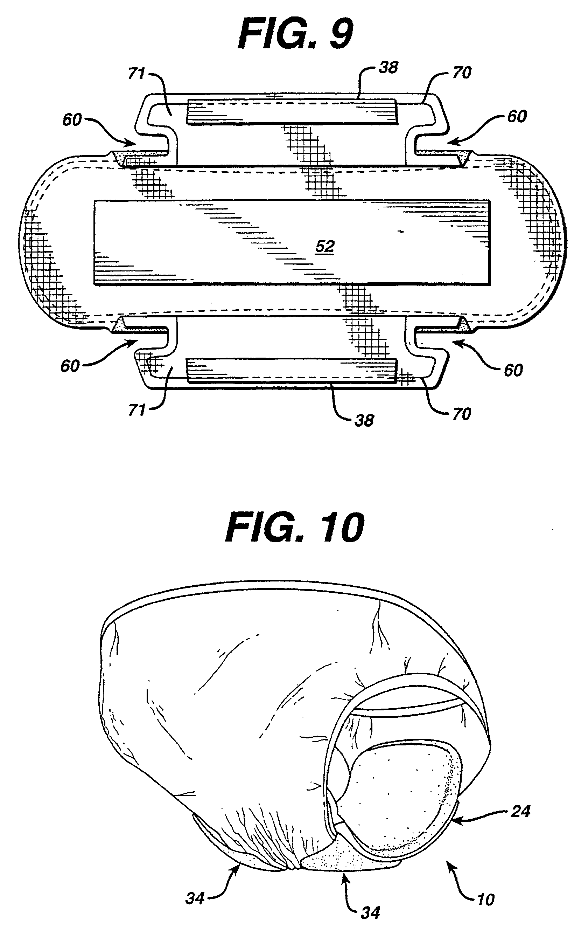

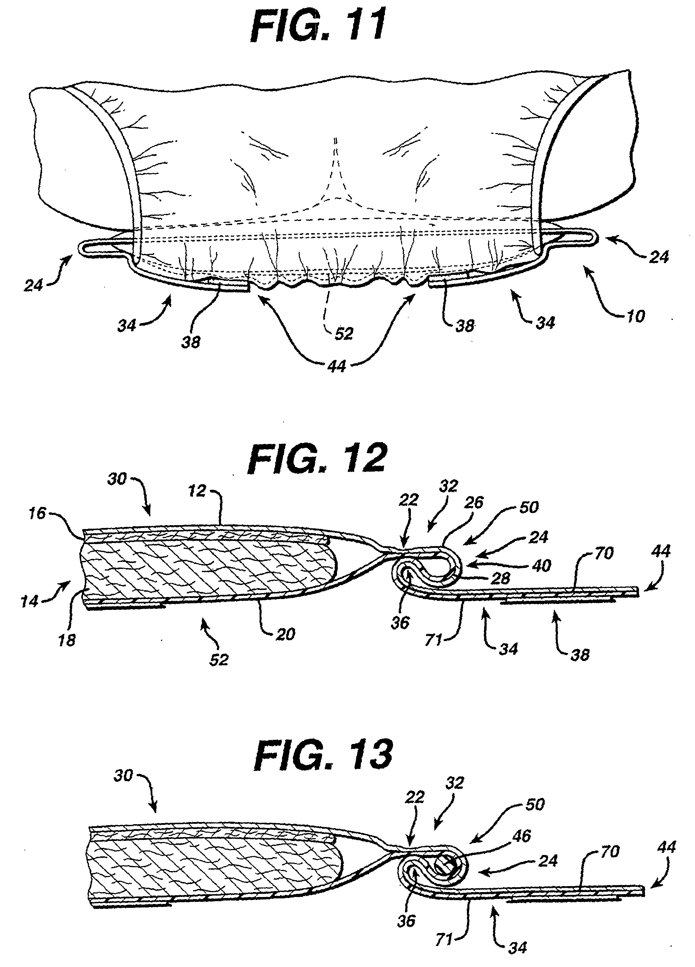

Sanitary napkin 10 further comprises a pair of panels 44 that extend laterally outward from the longitudinal edges of the napkin. Panels 24 are folded along the longitudinal side edges of the napkin under the garment-facing side of the sanitary napkin and are affixed to the barrier layer along a line of juncture which is inward from the longitudinal side edge of the sanitary napkin. The freely extending distal ends of the panels form outwardly extending flaps. Referring to Figures 10 and 11, in use, the sanitary napkin is placed in a crotch portion of an undergarment and the flaps are folded over the edges of a crotch portion of the undergarment whereupon the flaps maintain at least a portion of the edges of the crotch portion of the undergarment in a position which is inward from the longitudinal side edges of the sanitary napkin and underneath the main body, and preferably maintains at least a portion of the edges in a position underneath the absorbent system.

In order for the flaps to be attached to the wearer's undergarment in a comfortable manner, it is preferred that the flaps should not gather the undergarment in an amount which exceeds 25 percent of the original undergarment width as measured in the crotch region. More specifically, as is well known, the crotch region of an undergarment possesses a substantially parabolic shape wherein the center of the crotch is relatively narrow and then widens substantially in a direction towards both the front and rear panels of the undergarment. Thus, an absorbent article having flaps which are adapted to gather the edges of the crotch region of the undergarment towards the longitudinal centerline of the absorbent article will tend to gather the undergarment in those regions of the crotch which widen toward the front and rear of the undergarment. Accordingly, it is the transverse end regions of each flap (i.e. the corner regions of the flap adjacent the juncture zone which are folded around the edges of the wearer's undergarment) which will have the greatest tendency to alter or gather the normal edge of the undergarment inward towards the longitudinal centerline of the absorbent article. In accordance with the present invention, the distance between the flaps, as measured across the main body of the absorbent article from one juncture zone to an opposite juncture zone in a region proximate to the transverse ends of the flaps should not distort or gather the undergarment more than 25 percent of its original undistorted width, preferably less than 20 percent and most preferably less than 10 percent of the original undergarment width.

The flaps may be of a length sufficient to completely encircle the crotch portion of the undergarment or alternatively they may have a length which is sufficient to be adhesively secured to the garment side of the undergarment so as to gather the undergarment well within the boundary of the main body 30 of the sanitary napkin. As a result, the sides of the undergarment are retained under the sanitary napkin and thus shielded against wetting and/or staining.

The structure of each flap is best shown in Figures 2 and 3. Side panels originate from opposite respective side edges of the main body 30 and include longer base portion adjacent the longitudinal sides of the sanitary napkin which tapers towards the distal ends of the panel. The panels may include a cover layer portion 26, continuous with the cover layer 12, or a barrier layer portion 28, continuous with the barrier layer 20 or both a cover layer portion 26 and a barrier layer portion 28. Optionally, the cover layer portion 26 may comprise a separate non-woven fabric to provide a softer texture and to eliminate the "plastic" feel generally associated with the use of apertured polymeric films.

The cover layer portion 26 and barrier layer 28 are peripherally united to the barrier layer portion 28 by the seal line 22 (shown in Figure 3) adjacent the flange seal along the longitudinal sides of the napkin. The bond in seal line 22 may be made by means of adhesives, heat-bonding, ultrasonic bonding, radio frequency sealing, mechanical crimping, and the like and combinations thereof. Panel 24 may also comprise between cover layer portion 26 and barrier layer portion 28 additional material such as that of the absorbent core, the transfer layer, a fibrous highloft material, a resilient sheet of a polymeric foam material or combinations thereof.

Each panel 24 is comprised of a plurality of portions continuous with one another as hereinafter defined, a distal end 44 and a proximal end 32, defining therebetween a flap body portion 34 and a juncture zone 36 which is adjacent the proximal end 32. Proximal end 32 originates at a side edge toward a longitudinal centerline of the main body of the sanitary napkin. Juncture zone 36, defined between proximal end 32 and flap body portion 34 is in the form of a line substantially parallel to the side edge of main body 30. Intermediate proximal end 32 and flap body portion 34 is at least one folding axis 40 about which the panel is folded in an orientation under the barrier layer in a direction toward the longitudinal centerline of the sanitary napkin.

The panel 24 is affixed, at least in part, to the barrier layer 20 underneath the main body 30 at juncture zone 36 by means of adhesives, heat-bonding, ultrasonic bonding, radio frequency sealing, mechanical crimping, and the like and combinations thereof. The juncture zone may be a substantially straight line or alternatively may be in an arcuate shape either convex inward or convex outward with respect to the longitudinal centerline of the sanitary napkin.

The portion of the panel 24 between the proximal end 32 and the juncture zone 36 defines the looping member 50. The looping member 50 is substantially unitized. Where the flap is of a single layer construction, e.g. an extension of the cover layer, the looping member will generally be substantially unitized. Where the panel is of a multi-layer construction, it will be substantially unitized wherein the layers which form the looping member 50 are appreciably attached to one another. Any conventional method of attachment may be used. In Figures 3 - 6, the layers 26 and 28 are attached to one another over their entire facing-surface areas.

Flap body portion 34 is allowed to extend freely from the juncture zone 36 that is located inwardly of the respective side edge of the main body 30. Each of the flaps 34 have stiffening elements 70 which create lateral stiffness in at least a portion of the flap to provide a preferential bending line or hinge axis at the line of juncture of the flap. The stiffening elements 70 create stiffened regions 71 in the flap which provide enhanced control when a user folds the flaps around a crotch portion of an undergarment. More specifically, when a flap 34 has stiffening elements 70 throughout a substantial portion of the body of the flap and is wrapped around the panty elastic, it bends at a predetermined single hinge line. Due to the contour shape of an undergarment, the flap bends primarily in the longitudinally oriented direction, but also has some bending components in the transverse direction. As used herein, "bending in the longitudinal direction" refers to bending that occurs primarily in the longitudinal direction. In accordance with the present invention, the stiffened regions of the flap 34 provide greater control when folding the flaps 34 over the edges of the undergarment. In the present invention, the stiffening elements 70 create lateral stiffness in the flap but do not increase the longitudinal stiffness of the flap.

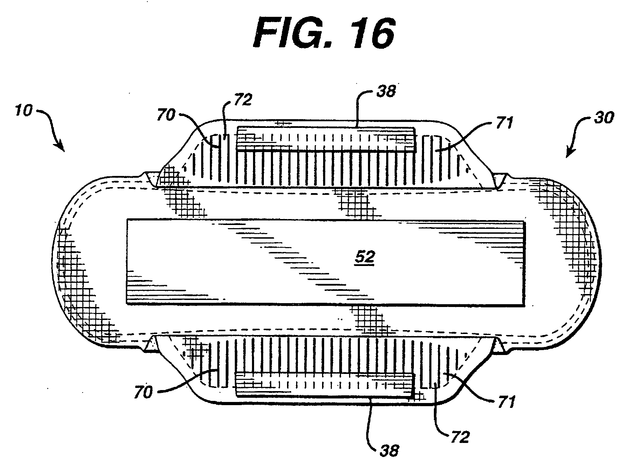

The stiffened portion 71 in the flap 34 covers substantially the entire body of the flap. The stiffened portion 71 need not extend fully to the edges of the flap, (including the opposite transverse edges as well as the distal edge 33 of the flap) and may be inset slightly from these edges provided of course that the flap maintains its enhanced lateral stiffness over a substantial portion of the flap. The stiffened portion 71 may terminate at the line of juncture in a substantially straight line, as illustrated in Figure 2, substantially parallel to the line of juncture 29 or preferably may terminate in an arcuate line, as illustrated in Figure 16, which allows the flap 34 to more easily conform to the arcuate shape of a crotch region of a wearer's undergarment. In a most preferred embodiment, the absorbent core has a substantially hourglass shape, i.e. wider transverse end regions 27 and narrower center regions with arcuate longitudinal sides stiffened portion 71 terminates in an arcuate line which is substantially parallel to the arcuate longitudinal sides of the absorbent core. The stiffened portion 71 preferably extends across the entire width of the flap. The stiffened portion 71 may be continuous or discontinuous, provided of course that any discontinuous regions do not provide a second preferential bending zone across the length of the flap.

The stiffened portion 71 of the flap 34 has a higher resistance to laterally compressive forces relative to the laminate of cover layer and barrier layer from which the flaps are formed. The stiffened portion 71 should have a resistance to lateral compression which is generally between 1.5 and 200 times greater that a resistance to lateral compression in the laminate of cover layer and barrier layer of the flap 34 and is preferably between 1.5 and 50 times greater and most preferably between 2 and 8 times greater. The relative stiffness or flexibility of a flap's resistance to lateral compression can be characterized by measuring its resistance to bending and can be conveniently determined with a Genuine Gurley TM Bending Resistance/Stiffness Tester, Model 4171 D, which is commercially available from Gurley Precision Instruments, Inc., Troy New York. When samples a stiffened portion 71 of the present flaps 34 were evaluated on this apparatus, a 2.52 centimeter by 5.04 centimeter sample had a resistance to bending in a range of from greater than 3 grams to about 250 grams, preferably from 4 grams to 50 grams, and most preferably from 5 grams to 20 grams.

Referring to Figure 16, according to the invention the stiffened portion 71 may comprise a plurality of individual stiffening elements 70 which are separated by one or more laterally extending flexible axes 72, such as, for example in a striped pattern wherein two or more stripes of stiffening element 70 create stiffened portion 71 which extends substantially across the length dimension of the flap. By separating the stiffened portions 71 by one or more laterally extending flexible axes 72 across the width of the flap, the distal region of the flap resists compression due to the application of laterally compressive forces, yet is flexible along the width of the flap (i.e. it would not inhibit longitudinal deformation of the sanitary napkin 1). This embodiment enables the sanitary napkin 1 to easily conform to a user's body when placed in an undergarment but provides resistance to laterally compressive forces. Thus, in accordance with this embodiment of the invention, the stiffened portion 71 of the flap is freely flexible across its width but resists bending across its length. In this manner, each flap not only preferentially bends at the line of juncture 29 and resists lateral bending in the stiffened portion 71 of the flap 34, but is also flexible in a longitudinal direction of the napkin.

The stiffened portion 71 of the flap 34 may be created by the incorporating into the flap any material which increases the stiffness of the flap relative to regions of the flap which to not contain this material. Examples of suitable materials include, but are not limited to tissue, non-woven fabric, polymer film, airlaid pulp, polymeric foam, non-pressure sensitive adhesive, embossments and the like and combinations thereof. The added materials are preferably flexible so as not to create discomfort to the user of the napkin and will generally range in thickness from about 0.02 millimeters to about 2 millimeters, preferably from about 0.025 millimeters (about 1 mil) to about 0.25 millimeters (about 10 mils) thick. While it is preferred that the materials incorporated into the flap be non-wicking, it is possible to use material which are ordinarily subject to wet collapse since they are not in contact with body fluids. It is also preferred that the materials incorporated into the flaps 34 be resilient. By providing resilience to the flaps 3, when the flaps 34 are folded under the user's undergarment and affixed to the undergarment in a central region of the absorbent napkin, they provide the napkin with greater recovery to lateral compressive forces. That is, since it is the central region of a sanitary absorbent napkin which is subjected to the laterally compressive forces of a user's thighs, the resilient material in the flaps 3, when folded and adhered under the napkin, enables the sanitary napkin 1 to better maintain its original shape and reduce bunching, twisting and roping.

As a result of the effective affixation point of the flap 34 being located inwardly of the longitudinal side edge in combination with the flap being stiffened, a number of advantages occur. When the flaps 34 are folded to encircle the crotch portion of the undergarment, and depending upon the width of the crotch portion of the undergarment, the flaps 34 gather or maintain the undergarment material between the two opposite juncture zones 36. As a result, the undergarment is confined well within the boundary of the main body 30 that shields that portion of the undergarment from wetting. In a preferred embodiment, the affixation points in the juncture zone are at least 71 mm apart as measured from one flap to an opposite flap, more preferably at least 75 mm apart and most preferably between 75 and 85 mm apart. When the juncture zone is in the form of a non-linear line, e.g. such as a convex curve, the distance between the affixation points should be determined at the opposite transverse end regions 70 and 71 of the flap.

In addition, since the effective affixation point of each flap 34 is located inwardly of the side edges of the absorbent article, this eliminates or substantially reduces the likelihood that the sides of the main body 30 will slope downwardly under the effect of tension communicated by the flaps. That is, if the tension vectors of the flaps act on the side edges of the absorbent article, it will be apparent that the main body will slope downwardly at the sides. Such downward sloping configuration is undesirable because it may induce liquid that has not immediately been absorbed through the cover layer 12 to leak sideways under the effect of gravity and unto the wearer's undergarment or garments. In accordance with the present invention, tension communicated to flaps which are attached inwardly of the longitudinal side edges of the absorbent article is not transmitted to the side edges and thus will not cause the sides of the main body to slope downwardly.

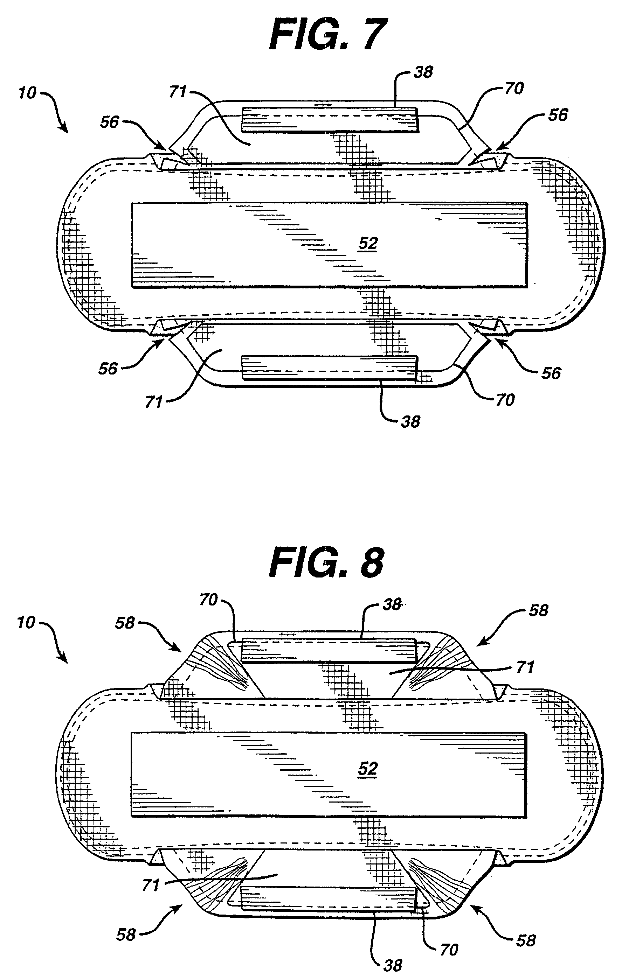

The base portion of each panel adjacent the proximal end 32 of each flap 34 is slightly shorter (dimension measured along a line transverse to the side edges of the main body 30) than the length of the main body. Preferably, the longitudinal dimension of the proximal end 32 is preferably from 50% to 90% of the length of the main body and is most preferably about 60% to 85% of the total length of the main body. The main body portion of the panel which forms the freely extending flaps in a region between the proximal portion and the distal portion tapers longitudinally from the base portion and is preferably from 25% to 75% of the length of the main body, most preferably between 30% to 50% of the length of the main body. For large flaps, it is preferred that the flap be longitudinally extensible to eliminate wrinkling and to permit the flaps to conformably adapt to the complex three dimensional shape of a crotch portion of a wearer's undergarment. In accordance with this aspect of the invention the flaps may comprise longitudinally elastic materials or as illustrated in Figure 8, may contain slits of pleats 58 which permit the flap to conform to the crotch portion of an undergarment.

The juncture zone 36 is inwardly displaced with relation to the side edge by a distance which may vary in accordance with the intended application, provided, of course, that it is sufficiently inward to maintain at least a portion of the crotch portion of an undergarment beneath the main body and inward from the longitudinal side edges of the sanitary napkin. In a preferred embodiment, this distance is in the range from about 1/8 inch to about ½ inch. Most preferably this distance is about 1/8 inch. In addition the shape of the juncture zone may also vary in accordance with the intended application. For example, the juncture zone may comprise a series of discrete points of affixation. Thus, a plurality of juncture zones may be formed along the flap to affix the flap to the barrier layer at a plurality of spaced apart locations. In accordance with this aspect of the invention, one or more separate juncture zones may be located intermediate the respective opposite longitudinal end regions of the flaps. Alternatively, the juncture zone may comprise a single continuous seal which extends from one longitudinal end region to an opposite longitudinal end region of the flap.

The continuous seal line may be linear or curved. In a preferred embodiment, the continuous seal line comprises a curve having a shape which is a convex outward arcuate line with respect to a longitudinal centerline of the absorbent article.

As illustrated in Figures 3 -6, the longitudinal dimension of the flaps 34 may optionally be such as to allow the flaps to overlap one another when they are folded about the undergarment. In order to retain the flaps in such overlapping condition they are provided with adhesive zones 38 on their barrier layer portions. In accordance with this aspect of the invention, when the flaps are folded and overlap one another the adhesive zone 38 on one flap is bonded to the cover layer portion of the other flap. Thus, the undergarment is completely encircled so it remains constantly in the gathered condition under the main body 30. It will also be appreciated that the positioning flaps also provide a stabilization function by preventing the main body 30 from becoming detached or moving freely with relation to the undergarment.

In an alternative embodiment hook and loop type fasteners (available under the brand VELCRO) may be used for connecting the flaps together. For example, the hook-type patch could be connected to the barrier layer portion of one flap while the loop-type patch is placed on the cover layer portion of the other flap.

As illustrated in Figs. 10 and 11 flaps 34 need not completely encircle the crotch portion of the undergarment in order to provide the desired undergarment gathering effect under the main body 30. For example, flaps 34 may be adhesively adhered to the wearer's undergarment, or may be designed with shape retentive properties so when they are bent under the undergarment they do not have a tendency to return to their original configuration.

The sanitary napkin design, described above, has the effect of pinching the undergarment in the region of the flaps 34 while allowing the undergarment, particularly near the longitudinal extremities of the main body 30 to fan out. Thus, the undergarment is shaped as a bow-tie, the narrowest portion of the undergarment being located in the central region where the flaps 34 are. The length of this narrowest zone can be controlled by varying the width of the flaps 34; the wider the flaps the longer the narrowest zone will be. Some applications, such as sanitary napkins for heavy menstrual flows, could benefit from wide flaps that would retain a larger portion of the undergarment under the main body 30 in comparison to a design using narrow flaps.

Referring to Figure 6, folding axis 40 is located laterally outward of proximal end 32 and juncture zone 36 thereby defining cavity 42. Cavity 42 may be sealed at opposite longitudinal end regions and filled with a fluid to provide a resilient, fluidly adaptive side edge margin. Suitable fluids include, but are not limited to one or a combination of the following fluidly adapting media: gases such as air, nitrogen and carbon dioxide, among others; liquids such as water and oils, among others, gels that are not too firm and than can flow in the fluidly adaptive component, and combinations of one or more of these media. The fluidly adaptive component may also contain, in addition to the fluid, some solid or semisolid substances or thixotropic gels. However, the nature and the amount of such substances should not be such as to prevent the fluid filled component from dynamically and transiently adapting to the contours of the user's body and clothing while the absorbent structure is being worn. It may be advantageous to use relatively high molecular weight which less easily diffuse through polymeric film materials.

Alternatively, or in addition to the presence of fluid in the cavity, as illustrated in Figure 4, one or more elastic elements 46 may be affixed to an interior surface of the cavity. In accordance with this embodiment, the juncture zone 36 is located laterally inward from the longitudinal side edges of the absorbent article to provide a cavity. The presence of the cavity along the longitudinal side edges of an absorbent article a gasketing effect between the absorbent article and the thighs of the wearer of the article. One or more elastic strips 46 may be placed in the cavity, wherein the elastic strips 46 extend longitudinally along the side edges of the absorbent article adjacent to the absorbent core. Each strip of elastic 46 is preferably in an elastically contracted position and secured to the inside surface of the cavity i.e. to the inner surface of the flange such that they maintain the side flanges in an upward body-facing orientation and thus gather the longitudinal sides of the absorbent article into a curved configuration. In accordance with this embodiment, the elastic strips 46 may extend the entire length of the absorbent article and may be secured to the cover layer 12, the barrier layer 20 or both at a plurality of bond sites along the length of the absorbent article. The expedient of incorporating elastic members 46 into the lateral margins of absorbent products is more fully disclosed in U.S. Patent Nos. 5,234,422 to Sneller et al., 5,074,856 to Coe et al., 5,032,121 to Mokry et al ., and 4,770,657 to Ellis et al.

Alternatively, as shown in Figure 5, a thin strip of absorbent material 48 may be placed in the cavity 42 to provide protective side cuffs along the longitudinal sides of the absorbent article. In a preferred embodiment, the thin strip of absorbent material 48 comprises a highloft, resilient fibrous material which is capable of absorbing and retaining fluid.

Referring to Figure 7, slits or notches 56 may be optionally provided in the side edges of the flap 34 near the proximal end 32. Occasionally, the adhesive portion 38 of a flap 34 may become unattached from the surface to which it was secured. In such a case the edges of the crotch portion of the undergarment of the wearer may no longer be maintained gathered under the napkin. They may cause the flap to ride up the leg of the wearer or may contain the flap itself within the undergarment, in either the case the undergarment may become soiled. Slits 56 serve as an aid to prevent this from occurring should the adhesive indeed become unattached, as they are positioned to trap and retain the elastic edge portion of the panties should the adhesive not be affixed. An alternative slit configuration wherein the slits 60 are rectangular in shape is illustrated in Figure 9.

In order to further enhance the stability of the sanitary napkin the main body 30 may be provided with adhesives, such as hot-melt adhesives capable of establishing a temporary bond with the undergarment material. These adhesives may be applied to the garment facing surface of the barrier layer 20 in various patterns, including complete adhesive coverage, parallel longitudinal lines, a line of adhesive following the perimeter of the structure, transverse lines of adhesive or the like. Alternatively, the sanitary napkin of this invention may be attached to a belt which encircles the waist of the wearer.

An important consideration when constructing a sanitary napkin 1 having flaps 34 with stiffening elements 70 is to determine where preferential fold line or hinge axis occurs relative to the central absorbent element. That is, the preferential fold line or hinge determines the ability of the flap to gather or maintain the edges of the panty towards the edges of the central absorbent element can be more easily controlled. Thus, the closer the two opposing preferential fold lines are together, the greater the tendency for the flaps 34 to gather the edges of the undergarment towards the longitudinal centerline 25 of the napkin. It is generally known in the art that an average undergarment has a medial crotch width of about 7.5 centimeters It is also known that the crotch region of an undergarment widens substantially towards the front and end regions. Thus, the distance between the preferential fold lines in the oppositely attached flaps 34 is preferably between about 7 centimeters and 8.5 centimeters It has been found that by providing the absorbent article with oppositely attached flaps 34 having stiffening elements 70 wherein the preferential fold lines in each respective flap are separated one from another by a distance of about 7 to 8.5 centimeters, that the flaps 34 easily gather the wider portions of the undergarment inward towards the longitudinal side edges 26 of the napkin and yet enables the flaps 34 to be secured around the side edges of the undergarment and adhesively secured under the absorbent element of the napkin rather than under an adjacent portion of the flap as illustrated in Figure 5. The expedient of providing stiffening elements 70 in the flaps 34 has also been found to aid in the ease of attachment, reduce wrinkles and creases in the flap and consequently helps the flap stay in place in a wearer's undergarment.

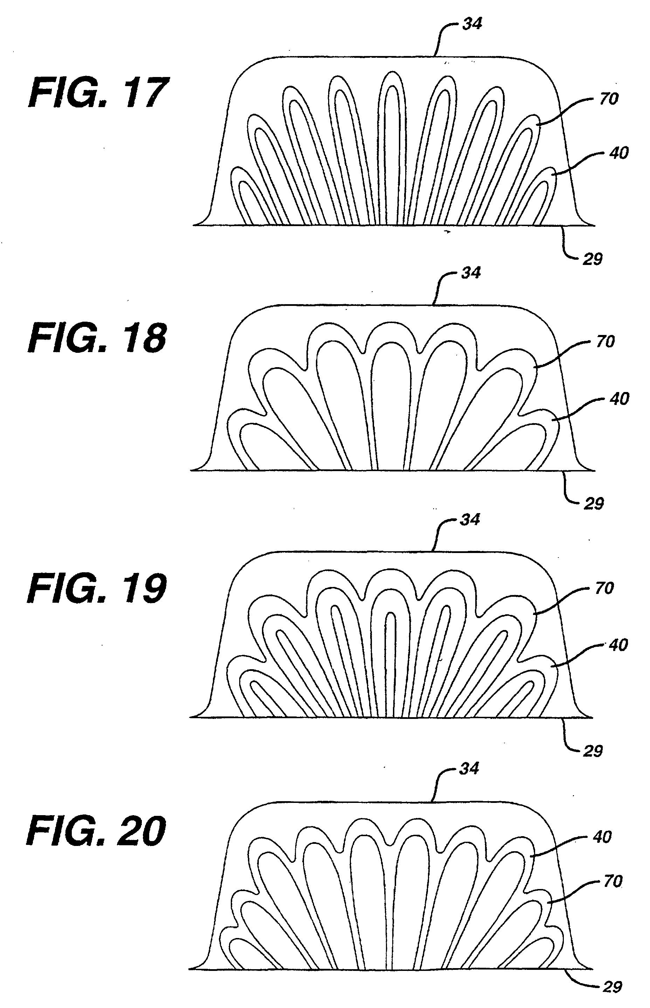

Referring to Figures 17-20, in another preferred embodiment of the present invention, the flaps 34 are provided with stiffening elements 70 which are created by embossing the flaps 34 in a pattern wherein the embossments 40 start at or slightly inward of the line of juncture 29 (inward from the line of juncture refers to extending into the looping member). The embossments 40 preferably terminate slightly inward from the distal edge of the flap, preferably from about 2 millimeters to about 10 millimeters inward from the distal edge of the flap. A preferred pattern of embossment is a series of loops 41 which start at or adjacent the line of juncture and which extend outward toward the distal edge of the flap and curve back toward the line of juncture where they terminate.

In another preferred embodiment of the present invention, the central absorbent core has a width which exceeds the width of a user's labia majora. While labial widths can vary widely from user to user, it has been found that an absorbent core width of at least 70 millimeters, preferably about 75 millimeters, generally exceeds the width of most women's labia majora. Since the medial crotch width of panties is generally about 7.5 centimeters. The flaps 34 of the present invention, when used on a sanitary napkin 1 having a core width of at least 70 millimeters and when used in a conventional panty, more easily folds around the edges of the panty crotch edge and have a greater ease of attachment than conventional sanitary napkins with flaps. As discussed above, since the line of juncture of the flap determines where the flap will fold, the flaps 34 can be adapted to assure that they are affixed to the underside of the user's undergarment in a location which is under the absorbent element rather than under an adjacent portion of the flap. A core width of at least 70 millimeters sufficiently separates the flexible portions 11 of the flaps 34 to reduce or eliminate the stresses which are applied to the flaps 34 by the side edges of a user's undergarment (which often contain an elastic element) and thereby provides the flaps 34 with an enhanced ability to remain attached to a wearer's undergarment during use.

In another embodiment of the present invention, the sanitary absorbent articles have an absorbent element formed by an upper body facing cover layer 5, a lower, garment facing barrier layer 10 and an absorbent core between the cover layer 5 and the barrier layer 10, as previously defined, wherein the absorbent element further comprises at least one conforming means. More specifically, the absorbent element has a central region, a first or anterior end region and a second or posterior end region. The central region has longitudinal edges coincident with the longitudinal edges of the absorbent element and first and second distal ends opposite each other defining an area that is sufficient to cover at least the woman's vestibule and labia majora in use. The first or anterior end region, extends from the first distal end of the central region and is adapted to cover at least a portion of the woman's mons pubis in use. The second or posterior end region extends from the second distal end of the central region and is adapted to cover at least a portion of the woman's posterior perineum in use. The absorbent element has at least one conforming means located within at least one of the end regions of the absorbent element, i.e. either in the first end region or in the second end region or both the first and second end regions. Referring again to Figure 1, the conforming means comprises a stiffened element 80 and a non-stiffened region 81. The non-stiffened region 81 is adjacent the distal end of the central region and between the central region and the stiffened element 80. The stiffened element 80 is located inward of the transverse and longitudinal edges of the absorbent element and between the non-stiffened region 81 and the distal end of the absorbent element. The non-stiffened region 81 extends transversely across the end region, generally perpendicular to the central longitudinal axis of the absorbent product from one longitudinal edge of the absorbent element to the opposite longitudinal edge of the absorbent element. The stiffened element 80 extends transversely across the end region, generally perpendicular to the central longitudinal axis of the absorbent product, at least at its intersection with that axis, and centrally occupies at least 50% of the width of the absorbent element. The stiffened element 80, in use, resists transverse bunching, i.e. it has a higher resistance to laterally compressive forces relative to the non-stiffened region 81. In combination, the stiffened element 80 and the non-stiffened region 81, in use, enables the end region of the absorbent product to preferentially bend longitudinally thereby providing the napkin with the ability to closely adapt to and fit the contours of a user's body. The stiffened element 80 may also act as a barrier to fluid wicking and guide the fluid so that it is retained within the confines of the absorbent element. The conforming means thereby provides an axis of bending, that coincides with a transverse axis of the napkin, a resistance to bending and compression orthogonal to that axis, i.e., along the longitudinal axis of the napkin and a relatively unconstrained region that may puff and cuff outward to fit and conform to the body. The stiffened element 80 may optionally be adapted to prevent leakage of fluid from the edges, as hereinafter described. The stiffened element 80 may of itself be a bending means, or may include a separate bending means, such as an embossed channel, or may comprise a projection, pleat, slit, hinge means or thinned area, that provides an axis of flexibility, coincident with the axis of bending of such bending means as well as providing stiffening and compression resistance orthogonal to the axis of bending.

The center region may optionally contain one or more stiffening means, such as extra absorbent material, a sphagnum-moss containing insert, an embossed channel having a component parallel to the central longitudinal axis, and combinations thereof. Embossed channels are preferably located between the central longitudinal axis and the longitudinal edge, provided of course that the stiffening means in the central region is spaced apart from the stiffened element 80 in the end region. The stiffening means maintains the center of the absorbent element in a relatively flat profile along the longitudinal axis, and resists bending of the absorbent element transversely to that axis, so as to effectively conform to the body in that region, resist transverse bending and bunching and thereby prevent leakage of fluid from the absorbent element. In a most preferred embodiment, the center region comprises additional absorbent material relative to the end regions of the absorbent element, the additional absorbent material having a pair of arcuate channels embossed therein to form a stiffened center region and conforming means located within both end regions of the absorbent element, the conforming means comprising an embossed channel. It is preferred that the width dimension of the flap be equal to or exceed the length of the embossed channel. A preferred embossed channel length is about 75 millimeters.

The absorbent articles of the present invention may be constructed by a process which includes the steps of:
- adhering an absorbent core to a surface of a fluid permeable cover layer wherein the cover layer has a width which is greater than the width of the absorbent core,
- covering the absorbent core with a fluid impervious barrier layer, wherein the barrier layer has a width substantially equivalent to the width of the cover layer,
- sealing the cover layer to the barrier layer around a peripheral edge margin of the absorbent core to form a flange seal,
- cutting the cover layer and the barrier layer outward of the flange seal in a pattern which provides a sanitary napkin having a pair of laterally extending flaps, one flap extending from each respective longitudinal side edge of the sanitary napkin, the flaps being integrally formed from the cover layer and the barrier layer, the flaps having a proximal end adjacent each respective longitudinal side edge of the sanitary napkin and a distal end extending laterally outward from the respective longitudinal side edge of the sanitary napkin,
- folding the flaps along a folding axis under the barrier layer, and
affixing the flaps to the barrier layer along a juncture zone which is inward of the longitudinal side edges of the sanitary napkin such that the distal end of the flaps extend laterally inward toward a longitudinal centerline of the sanitary napkin.

The particular order of the above described process is not, per se, critical provided of course that the final product comprises an upper fluid permeable layer, a lower barrier layer and an absorbent layer between the upper layer and the lower layer. Accordingly, the step of affixing the flaps to the barrier layer may be performed prior to the step of cutting the cover and barrier layers. Similarly, the absorbent core may be adhered to the barrier layer prior to the cover layer. Thus, the only critical step in the present method is the expedient of affixing the integrally formed flaps to the barrier layer at the juncture zone. Most preferably, the folding axis is an imaginary line parallel to a longitudinal side edge of the sanitary napkin and approximately centrally located within the flange seal and wherein the juncture zone affixes the flaps to at least a portion of the flange seal.

Applications of the product and process of the present invention for sanitary and other health-care uses can be accomplished by any sanitary protection, incontinence, medical and absorbent methods and techniques known to those skilled in the art.

## Claims

1. A sanitary absorbent article, comprising:
(A) a main body (30) having opposed longitudinal side edges and a longitudinal centerline, said main body (30) including:
(i) a body-facing fluid-permeable upper layer (12) intended for placement against a perineal region of a wearer,
(ii) a garment-facing fluid-impervious lower layer (20), and
(iii) an absorbent layer (18) between the upper layer (12) and the lower layer (20);
(B) at least one panel (24) integrally formed from the upper layer (12), the lower layer (20) or a combination of both the upper layer (12) and the lower layer (20), said panel (24) extending from a longitudinal side edge of said main body (30), said panel (24) further including:
(i) a proximal end region (32) adjacent to the longitudinal side edge of said main body (30),
(ii) a distal end region (44) extending laterally from the longitudinal side edge of the sanitary absorbent article, the proximal end region (32) and the distal end region (44) defining therebetween a body portion,
(iii) a juncture zone (36) within the body portion and adjacent the proximal end region (32) of said panel (24), the juncture zone (36) attaching a portion of said panel (24) to the barrier layer (20) inwardly from the longitudinal side edge of said main body (30) thereby defining a freely extending flap (34),
(iv) a looping member (50) defined by the portion of the body portion (30) of said panel (24) between the proximal end region (32) and the juncture zone (36), the looping member (50) being substantially unitized;
said flap (34) being capable of being folded about an edge of an undergarment in a crotch portion thereof; and
when said portion of said flap (34) is in a folded condition about the edge of the undergarment said flap (34) providing means for holding at least a portion of the edge of the undergarment inwardly of said longitudinal side edge; **characterized by**
(C) the flap (34) having at least one stiffening element (70) which creates lateral stiffness without increasing longitudinal stiffness in at least a portion of the flap (34) to provide a preferential bending line (29, 40) at the juncture zone (36) of the flap (34) and the article, the flap (34) being capable of being folded about the preferential bending line (29, 40) onto itself at the juncture zone (36) such that the proximal end region (32) of the panel (24) projects in a direction generally transverse to said longitudinal side edge laterally inward towards the longitudinal centerline of said main body (30) on a garment-facing side of the sanitary absorbent article.

2. A sanitary absorbent article as defined in claim 1, wherein the continuous line of attachment is arcuate having a convex inward orientation with respect to the longitudinal centerline of the main body (30).

3. A sanitary absorbent article as defined in claim 1, wherein the juncture zone (36) of each panel (24) is located underneath the absorbent layer (18).

4. A sanitary absorbent article as defined in claim 1, wherein the juncture zone (36) of each panel (24) is located underneath a lower layer around their respective peripheral edge margins.

5. A sanitary absorbent article as defined in claim 4, wherein the folding axis (40) is adjacent to a flange seal (22) and the juncture zone (36) is located laterally inward of the respective folding axis of each panel (24) thereby defining a cavity (42) along the longitudinal edges of said main body (30).

6. A sanitary absorbent article as defined in claim 5, wherein the juncture zone (36) extends longitudinally along the entire length of the flange seal (22) and wherein the cavity (42) extends along the longitudinal sides of said main body (30).

7. A sanitary absorbent article as defined in claim 6, wherein the cavity (42) contains an elastic member (46), the elastic member (46) being in tension and secured along its length to the flange seal (22).

8. A sanitary absorbent article as defined in claim 1, wherein one of said flaps (34) includes an adhesive zone (38) located on a side of the flap (34) that faces the undergarment when said flap (34) is folded about the undergarment, the adhesive zone(38) being capable of establishing a bond with a side of the other of said laps (34) that faces away from the undergarment when said other flap (34) is folded about the undergarment.

9. A sanitary absorbent article as defined in claim 1, wherein the sanitary absorbent article is a sanitary napkin.

## Patentansprüche

1. Sanitärer Absorptionsgegenstand, mit:
(A) einem Hauptkörper (30) mit zueinander gegenüberliegenden, longitudinalen Seitenrändem und einer longitudinalen Mittellinie, wobei der Hauptkörper (30) umfasst:
(i) eine körperzugewandte, fluiddurchlässige obere Schicht (12) zum Anlegen an einen perenialen Bereich.eines Trägers,
(ii) eine kleidungszugewandte, fluidundurchlässige untere Schicht (20) und
(iii) eine absorbierende Schicht (18) zwischen der oberen Schicht (12) und der unteren Schicht (20);
(B) zumindest einer Lasche (24), die einstückig aus der oberen Schicht (12), der unteren Schicht (20) oder einer Kombination von beiden, der oberen Schicht (12) und der unteren Schicht (20), ausgebildet ist, wobei sich die Lasche (24) von einem longitudinalen Seitenrand des Hauptkörpers (30) aus erstreckt und weiterhin umfasst:
(i) einen proximalen Endbereich (32) benachbart zu dem longitudinalen Seitenrand des Hauptkörpers (30),
(ii) einen distalen Endbereich (44), der sich lateral ausgehend von dem longitudinalen Seitenrand des sanitären Absorptionsgegenstandes erstreckt, wobei der proximale Endbereich (32) und der distale Endbereich (44) dazwischen einen Körperabschnitt definieren,
(iii) eine Verbindungszone (36) innerhalb des Körperabschnittes und benachbart zum proximalen Endbereich (32) der Lasche (24), wobei die Verbindungszone (36) einen Abschnitt der Lasche (24) an der Sperrschicht (20) innerhalb des longitudinalen Seitenrands des Hauptkörpers (30) befestigt, wodurch ein sich frei erstreckender Flügel (34) definiert wird,
(iv) ein Windungsteil (50), das durch den Abschnitt des Körperabschnitts (30) der Lasche (24) zwischen dem proximalen Endbereich (32) und der Verbindungszone (36) definiert ist, wobei das Windungsteil (50) im wesentlichen eine Einheit bildet;
wobei der Flügel (34) um einen Rand eines Unterbekleidungsstücks in einem Schrittabschnitt desselben faltbar ist; und
wobei der Flügel (34), wenn der Abschnitt des Flügels (34) in einem um den Rand des Unterbekleidungsstücks gefalteten Zustand ist, Mittel zum Halten von zumindest einem Abschnitt des Randes des Unterbekleidungsstücks innerhalb des longitudinalen Seitenrands bereitstellt;
**dadurch gekennzeichnet, dass**
(C) der Flügel (34) mit zumindest einem Verstärkungselement (70) versehen ist, das eine laterale Steifigkeit erzeugt, ohne die longitudinale Steifigkeit in zumindest einem Abschnitt des Flügels (34) zu erhöhen, um eine Vorzugskrümmungslinie (29, 40) an der Verbindungszone (36) des Flügels (34) und des Gegenstandes bereitzustellen, wobei der Flügel (34) um die Vorzugskrümmungslinie (29, 40) auf sich selber an der Verbindungszone (36) faltbar ist, so dass der proximale Endbereich (32) der Lasche (24) sich auf einer kleidungszugewandten Seite des sanitären Absorptionsgegenstands in einer Richtung erstreckt, die im wesentlichen quer zu dem longitudinalen Seitenrand und lateral innerhalb der longitudinalen Mittellinie des Hauptkörpers (30) verläuft.

2. Sanitärer Absorptionsgegenstand nach Anspruch 1, **dadurch gekennzeichnet, dass** die durchgängige Befestigungslinie bogenförmig ist mit einer konvexen Orientierung nach innen bezüglich der longitudinalen Mittellinie des Hauptkörpers (30).

3. Sanitärer Absorptionsgegenstand nach Anspruch 1, **dadurch gekennzeichnet, dass** sich die Verbindungszone (36) jeder Lasche (24) unterhalb der Absorptionsschicht (18) befindet.

4. Sanitärer Absorptionsgegenstand nach Anspruch 1, **dadurch gekennzeichnet, dass** sich die Verbindungszone (36) jeder Lasche (24) unterhalb einer unteren Schicht um ihre jeweiligen peripheren Randbegrenzungen herum befindet.

5. Sanitärer Absorptionsgegenstand nach Anspruch 4, **dadurch gekennzeichnet, dass** die Faltungsachse (40) benachbart zu einer Flanschdichtung (22) ist und dass sich die Verbindungszone (36) lateral innerhalb der jeweiligen Faltungsachse jeder Lasche (24) befindet, wodurch ein Hohlraum (42) entlang der longitudinalen Ränder des Hauptkörpers (30) definiert wird.

6. Sanitärer Absorptionsgegenstand nach Anspruch 5, **dadurch gekennzeichnet, dass** sich die Verbindungszone (36) longitudinal entlang der gesamten Länge der Flanschdichtung (22) erstreckt und dass sich der Hohlraum (42) entlang den longitudinalen Seiten des Hauptkörpers (30) erstreckt.

7. Sanitärer Absorptionsgegenstand nach Anspruch 6, **dadurch gekennzeichnet, dass** der Hohlraum (42) ein elastisches Teil (46) enthält, wobei das elastische Teil (46) unter Spannung steht und entlang seiner Länge an der Flanschdichtung (22) befestigt ist.

8. Sanitärer Absorptionsgegenstand nach Anspruch 1, **dadurch gekennzeichnet, dass** einer der Flügel (34) eine Haftzone (38) aufweist, die sich auf einer Seite des Flügels (34) befindet, die dem Unterbekleidungsstück zugewandt ist, wenn der Flügel (34) um das Unterbekleidungsstück gefaltet ist, wobei die Haftzone (38) fähig ist, eine Verbindung mit einer Seite des anderen der beiden Flügel (34) herzustellen, die von dem Unterbekleidungsstück abgewandt ist, wenn der andere Flügel (34) um das Unterbekleidungsstück gefaltet wird.

9. Sanitärer Absorptionsgegenstand nach Anspruch 1, **dadurch gekennzeichnet, dass** der sanitäre Absorptionsgegenstand eine Binde ist.

## Revendications

1. Article sanitaire absorbant comprenant :
(A) un corps principal (30) possédant des bords latéraux longitudinaux opposés et une ligne centrale longitudinale, ledit corps principal (30) comprenant :
(i) une couche supérieure (12) perméable aux fluides faisant face au corps, destinée à être placée contre la région périnéale d'un porteur,
(ii) une couche inférieure (20) imperméable aux fluides faisant face au vêtement, et
(iii) une couche absorbante (18) entre la couche supérieure (12) et la couche inférieure (20) ;
(B) au moins un panneau (24) intégralement formé à partir de la couche supérieure (12), la couche inférieure (20) ou une combinaison de la couche supérieure (12) et de la couche inférieure (20), ledit panneau (24) s'étendant à partir d'un bord longitudinal latéral dudit corps principal (30), ledit panneau (24) comprenant en outre :
(i) une région d'extrémité proximale (32) adjacente au bord longitudinal latéral dudit corps principal (30),
(ii) une région d'extrémité distale (44) s'étendant latéralement à partir du bord longitudinal latéral de l'article sanitaire absorbant, la région d'extrémité proximale (32) et la région d'extrémité distale (44) définissant entre les deux une partie de corps,
(iii) une zone de jonction (36) à l'intérieur de la partie de corps et adjacente à la région d'extrémité proximale (32) dudit panneau (24), la zone de jonction (36) attachant une partie dudit panneau (24) à la couche barrière (20) vers l'intérieur par rapport au bord longitudinal latéral dudit corps principal (30), définissant ainsi une patte (34) s'étendant librement,
(iv) un élément en boucle (50) défini par la partie de la partie de corps (30) dudit panneau (24) comprise entre la région d'extrémité proximale (32) et la zone de jonction (36), l'élément en boucle (50) étant sensiblement égalisé ;
ladite patte (34) pouvant être repliée sur un bord d'un sous-vêtement dans une partie de l'entrejambe de celui-ci ; et
lorsque ladite partie de ladite patte (34) est dans un état replié sur le bord du sous-vêtement, ladite patte (34) possédant des moyens pour tenir au moins une partie du bord du sous-vêtement vers l'intérieur dudit bord longitudinal latéral ; **caractérisé en ce que**
(C) la patte (34) possède au moins un élément de renfort (70) qui crée une rigidité latérale sans augmenter la rigidité longitudinale dans au moins une partie de la patte (34) pour fournir une ligne de flexion préférentielle (29, 40) à la zone de jonction (36) de la patte (34) et de l'article, la patte (34) pouvant être repliée sur elle-même par la ligne de pliure préférentielle (29, 40) au niveau de la zone de jonction (36) de manière à ce que la région d'extrémité proximale (32) du panneau (24) s'avance dans une direction généralement transversale par rapport audit bord longitudinal latéral et latéralement vers l'intérieur, en direction de la ligne centrale longitudinale dudit corps principal (30) sur un côté de l'article sanitaire absorbant faisant face au vêtement.

2. Article sanitaire absorbant selon la revendication 1, dans lequel la ligne continue d'attache est arquée, possédant une orientation convexe vers l'intérieur relativement à la ligne centrale longitudinale du corps principal (30).

3. Article sanitaire absorbant selon la revendication 1, dans lequel la zone de jonction (36) de chaque panneau (24) est située en dessous de la couche absorbante (18).

4. Article sanitaire absorbant selon la revendication 1, dans lequel la zone de jonction (36) de chaque panneau (24) est située en dessous d'une couche inférieure, autour des lisières périphériques respectives de leurs bords.

5. Article sanitaire absorbant selon la revendication 4, dans lequel l'axe de pliure (40) est adjacent à un scellement de bride (22) et la zone de jonction (36) est située latéralement vers l'intérieur par rapport à l'axe de pliure respectif de chaque panneau (24), définissant ainsi une cavité (42) le long des bords longitudinaux dudit corps principal (30).

6. Article sanitaire absorbant selon la revendication 5, dans lequel la zone de jonction (36) s'étend longitudinalement le long de toute la longueur du scellement de bride (22) et dans lequel la cavité (42) s'étend le long des côtés longitudinaux dudit corps principal (30).

7. Article sanitaire absorbant selon la revendication 6, dans lequel la cavité (42) renferme un élément élastique (46), l'élément élastique (46) étant en tension et fixé le long de sa longueur au scellement de bride (22).

8. Article sanitaire absorbant selon la revendication 1, dans lequel l'une desdites pattes (34) comprend une zone adhésive (38) située sur un côté de la patte (34) qui fait face au sous-vêtement lorsque ladite patte (34) est repliée sur le sous-vêtement, la zone adhésive (38) pouvant établir un lien avec un côté de l'autre desdites pattes (34) qui ne fait pas face au sous-vêtement lorsque ladite autre patte (34) est repliée sur le sous-vêtement.

9. Article sanitaire absorbant selon la revendication 1, dans lequel l'article sanitaire absorbant est une serviette sanitaire.
